# EUROPEAN PATENT APPLICATION

(11) **EP 3 431 173 A1**
(43) Date of publication of application: **23.01.2019**
(21) Application number: 17182070.7
(22) Date of filing: 19.07.2017
(51) Int. Cl.: B01J 4/02, B01J 19/24, A61K 48/00, A61K 47/68

(54) **CONTINUOUS MANUFACTURE OF GUIDANCE MOLECULE DRUG CONJUGATES**

(71) Applicant: Bayer Pharma Aktiengesellschaft, 13353 Berlin (DE)
(72) Inventor: Paulsen, Holger, 40724 Hilden (DE); Krüger, Joachim, 40489 Düsseldorf (DE); Kaiser, Klaus, 51467 Bergisch Gladbach (DE); Lingen, Verena, 50733 Köln (DE); Gottfried, Michael, 42349 Wuppertal (DE)
(74) Representative: BIP Patents

(57) **Abstract**

Described herein is a modification unit (37) for the continuous, germ-reduced processing of a guidance molecule and a linker comprising the following components:
- at least one reservoir containing the guidance molecule in buffer solution (1) and/or at least one inlet for a product stream containing the guidance molecule in buffer solution,
- at least one reservoir containing the linker in solution (2),
- at least one mixing device (3),
- at least two valves (7, 8), one for dosage of the guidance molecule and one for dosage of the linker molecule
- at least one outlet for the product stream comprising the guidance molecule-linker complexes and/or a reservoir for taking up the guidance molecule-linker complexes (5).

## Description

Antibody drug conjugates (ADCs) comprise antibodies attached to a biologically active e.g. cytotoxic substance. As therapeutics ADCs combine the exquisite specificity of antibodies, enabling discrimination between healthy and diseased tissue, with the cell-killing ability of cytotoxic drugs. In other words, the antibody is for example used to transport a cytotoxic substance directly to a diseased - e.g. a cancer - cell and release it inside the cell. In this way the healthy cells are largely spared the toxic side-effects that occur with traditional chemotherapy.

In detail, ADCs consist of three different components: the antibody, an active substance - e.g. drug such as a cytotoxic active ingredient also termed toxophore - and a linker connecting the antibody and the active substance. The antibody recognizes and binds to certain protein molecules e.g. tumor markers on the surface of tumor cells. This binding process triggers a mechanism, which transports the ADC inside the cell, where the active substance is separated from the antibody and can act on the cell for example via blocking important cell functions thereby leading to programmed cell death (apoptosis). Alternatively, the antibody recognizes and binds to said protein molecules e.g. tumor markers on the surface of tumor cells and the active substance acts on the tumor cells.

It is essential for the success of a therapy with ADCs that the conjugate does not lose its conjugated active substance as its passes through the body, and does not release it until it is inside the diseased cell. Moreover, the antibody has to specifically target the diseased cell. Therefore, it is desirable that the process of coupling of the biologically active substance via the linker affects the antibody as little as possible and delivers comparable ADC during scale up and manufacturing.

Hence, as the demand for ADCs is growing a more flexible production processes, which is easy to scale up, but takes *inter alia* the above mentioned particulars of the ADC system into account would be advantageous.

Therefore, it was an object of the present invention to provide more flexible production processes which is reliably, robust and reproducible under various process conditions as well as easy to scale up and does not affect the antibody.

This object is achieved via providing a modification unit (37) for the continuous, germ-reduced processing of a guidance molecule and a linker comprising the following components:
- at least one reservoir containing the guidance molecule in buffer solution (1) and/or at least one inlet for a product stream containing the guidance molecule in buffer solution
- at least one reservoir containing the linker in solution (2)
- at least one mixing device (3)
- at least two valves (7, 8), one for dosage of the guidance molecule and one for dosage of the linker molecule
- at least one outlet for the product stream comprising the guidance molecule-linker complexes and/or a reservoir for taking up the guidance molecule-linker complexes (5).

This modification unit enables a continuous attachment of the linker to the guidance molecule while maintaining an excellent stability of the guidance molecule. This finding is surprising since - without wishing to be bound by theory - it was expected that the guidance molecule would interact negatively with the large surface area present in the continuous process compared to a batch process. The large surface area is generated by the comparatively small tubing and the comparably small mixing devices and it was expected as the surface area increases also a larger percentage of guidance molecules would interact with said larger surface area which would negatively affect the quality of the guidance molecule in particular the aggregate status.

Moreover this unit is very easy to scale up to production scales i.e. to increase the total amount of product e.g. amount of ADC g/per week. This is the case since in order to achieve larger product quantities simply more starting material has to be provided and overall process duration will increase. However, it is neither necessary to build a larger production plant/facility, to validate another production plant/facility nor to undertake extensive comparability studies as is required for biologicals

It is to be understood that the terminology used herein is for the purpose of describing particular embodiments only, and is not intended to be limiting. As used in this specification and the appended claims,, the singular forms "a," "an," and "the" include plural referents unless the content clearly dictates otherwise. Thus, for example, reference to "a device" includes a combination of two or more such devices. "

As used herein the term "guidance molecule" refers to an entity capable of finding and/or interacting with a specific target.

Examples of guidance molecules are peptides, proteins and nucleic acids such as DNA and RNA molecules including RNAi molecules.

As used herein the term "peptide" refers to a polymer of amino acids of relatively short length (e.g. less than 50 amino acids). The polymer may be linear or branched, it may comprise modified amino acids, and it may be interrupted by non-amino acids. The term also encompasses an amino acid polymer that has been modified; for example, by disulfide bond formation, glycosylation, lipidation, acetylation, phosphorylation, or any other manipulation, such as conjugation with a labeling component, such as but not limited to, fluorescent markers, particles, biotin, beads, proteins, radioactive labels, chemiluminescent tags, bioluminescent labels, and the like.

As used herein the term "protein" refers to a polypeptide of amino acids. The term encompasses proteins that may be full-length, wild-type, or fragments thereof. The protein may be human, non- human, and an artificial or chemical mimetic of a corresponding naturally occurring amino acid, as well as to naturally occurring amino acid polymers and non- naturally occurring amino acid polymer. Moreover, also encompassed are proteins in which specific amino acids are exchanged e.g. to enable a "site-specific-conjugation" of the active substance.

As used herein, the term "nucleic acid" refers to deoxyribonucleotides or ribonucleotides and polymers thereof in either single- or double-stranded form. Unless specifically limited, the terms encompass nucleic acids containing analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g. degenerate codon substitutions) and complementary sequences as well as the sequence explicitly indicated. Moreover, also encompassed are nucleic acids, in which specific base pairs are exchanged e.g. to enable a "site-specific-conjugation" of the active substance.

In a preferred embodiment the guidance molecule is an antibody and/or an antigen-binding fragment.

The term "antibody" as used herein refers to a binding molecule such as an immunoglobulin or immunologically active portion of an immunoglobulin, i.e., a molecule that contains an antigen-binding site.

As used herein the term "antibody-drug-conjugate (ADC)" refers to a complex comprising at least one antibody, at least one drug and at least one 'linker' which connects the antibody with the drug.

As used herein the term "antigen-binding antibody fragment" or "antigen-binding fragment" refers to a fragment of an antibody/immunoglobulin (e.g. the variable domains of an IgG) which still comprise the antigen binding domains of the antibody/immunoglobulin. The "antigen binding domain" of an antibody typically comprises one or more hypervariable regions of an antibody.

The terms "biologically active substance", "active substance", "active agent", "drug," or "therapeutic," as used herein refer to any atom and/or molecule, molecular complex or substance administered to an organism for diagnostic or therapeutic purposes, including the treatment of a disease or infection, medical imaging, monitoring, contraceptive, cosmetic, nutraceutical, pharmaceutical and prophylactic applications. The term "drug" includes any such atom and/or molecule, molecular complex or substance that is chemically modified and/or operatively attached to a biologic or biocompatible structure. The term "prodrug" refers to a drug, drug precursor or modified drug that is not fully active or available until converted in vivo to its therapeutically active or available form.

The term "toxophore" as used herein refers to a chemical group that produces a toxic effect when administered to a cell.

In other words, the active substance can for example be an atom such as a thorium atom or an antimitotic agent.

As used herein the term "unit" or "unit operation" refers to a device or a combination of devices that perform one process step in a production process of a guidance molecule such as an antibody and/or an antibody drug conjugate. In other words, in order to provide the final a guidance molecule i.e. the product said product will have to pass several units.

Since the step of attaching the linker to the guidance molecule is generally referred to as modification, the device enabling this attachment is termed modification unit.

As used herein, the term "continuous" refers to the fact that the input of the components to be processed and/or a product stream into a unit e.g. the modification unit and the removal of the processed components and/or the product stream from said unit, e.g. the modification unit, take place without interruption. In other words, a subsequent unit operation can start processing the product stream before a first unit operation has finished processing the product stream.

As used herein, the term "batchwise" refers to the fact the usually individual production cycles or production steps are handled discontinuously in a batchwise manner, with the entire product being collected and pooled prior to starting the next production step or the entire product being removed after completion of a production cycle. To produce again, it is then necessary to start a separate new product cycle/ batch.

The highly regulated pharmaceutical production requires great effort in terms of time, technology and personnel to provide cleaned and sterilized facilities in order to ensure a sterile product. Moreover, to reliably avoid cross-contamination in the event of a product changeover in a multipurpose system or between two product batches a complex cleaning validation is mandatory, which again requires great effort in terms of time and personnel. Thus, a continuous process for the production of ADCs is advantageous. This continuous process for the production of ADCs also has the advantage that it enables a safer handling of the highly potent active substance, e.g. a toxophore, via the closed system with small amounts of reaction volumes compared to batch operations with large volumes of highly potent active substance.

As used herein the term "pathogen-reduced" is used interchangeable with "microbe-reduced" and "germ-reduced" and refers to a state of reduced pathogenic count, including a reduced viral count, i.e. a pathogenic count per area or volume unit of close to zero that is achievable by means of a suitable germ-reducing method, wherein this germ-reducing method can be selected from gamma irradiation, beta irradiation, autoclaving, Ethylene Oxide (ETO) treatment, Ozone treatment, "Steam-In-Place" (SIP) and/or Heat in Place treatment or treatment with sanitization agent like 1 M NaOH. In other words, herein the terms "pathogen-reduced", "microbe-reduced" and "germ-reduced" also refer to a bioburden controlled status.

As used herein the term "product stream" refers to the fluid comprising the guidance molecule passing the unit for ultrafiltration and purification described herein. In other words, prior to entering the unit for ultrafiltration and purification the product stream can also be referred to as feed and upon leaving the unit for ultrafiltration and purification the product stream can also be referred to as retentate.

As used herein the term "reservoir" refers to a storage container such as a surge bag comprising components required in the process, e.g. buffers, the antibody, the linker or the active substance.

As used herein the term "mixing device" refers to an apparatus or a mechanism for rendering a heterogeneous physical system more homogeneous. In order to achieve a thorough mixing, devices with small spaces are preferred.

Examples of suitable mixing devices are a T-piece, a microblade mixer and static mixer with different mixing principles e.g. slit or helical static mixers.

As used herein the term "linker" refers to a molecule bound to the antibody and the active substance. Thus, the term "linker" describes non-cleavable and cleavable linker as well as chelators.

In addition to the advantages mentioned above it was surprisingly found that the modification unit facilities generation of a stable and consistent linker-antibody ratio. This is important since it facilitates the generation of a stable and consistent drug-antibody ratio (DAR). Only a stable and consistent number of molecules of the biologically active substance attached per antibody ensures comparable in vivo activity.

In one embodiment the modification unit described herein comprises at least three valves. Preferably, the third valve is used for controlling the inlet into the plug flow reactor.

It should be noted that the modification unit described herein may comprise more than three valves. A person skilled in the art knows how many valves are required for a given unit.

In one embodiment the modification unit described herein further comprises at least one residence time device

As used herein the term "residence time device" refers to a device ensuring a defined residence time, i.e. ensuring that after mixing the guidance molecules and the linker molecules always spend a similar amount of time in a continuous process. This has the effect that the consistent properties of the produced guidance molecule-linker molecule complexes can be easily ensured.

However, consistent properties of the produced guidance molecule-linker molecule complexes can also be achieved via choosing a suitable mixing device.

In a preferred embodiment the residence time device is a plug flow reactor.

In a preferred embodiment each portion of the product stream passes the residence time device after it has passed the mixing device. In one example of this embodiment each portion of the product stream passes a plug flow reactor after it has passed the mixing device.

As used herein the term "plug flow reactor" refers to a device in which the fluid is mixed in the radial direction but not in the axial direction (forwards or backwards). As it flows in the plug flow reactor the residence time of each portion of the fluid stream is a function of its position in the reactor, i.e. the residence time of the product stream in the plug flow reactor is narrow. In other words, ideally any part of the fluid stream spends the same amount of time in the plug flow reactor as any other part of the fluid stream.

An example of a plug flow reactor is a coiled flow inverter.

In a preferred embodiment the residence time device is a coiled flow inverter (CFI).

The CFI was introduced by Klutz et al. as a tool for continuous low pH viral inactivation (Klutz, S. et al. 2015, Journal of Biotechnology 213, pp. 120-130*).*

Alternatively, a continuous stirred tank reactor could be used instead of or in addition to a CFI.

If a continuous stirred tank reactor is used, the modification unit described herein further comprises an outflow.

In a preferred embodiment the modification unit is disposable and/or for single use. This has the advantage that numbering up in order to increase yield per time can be achieved much easier using disposable equipment than with traditional plants relying on stainless steel equipment, with complex cleaning infrastructure e.g. because also the cleaning infrastructure needs to be numbered up for stainless steel equipment.

In one embodiment the modification unit described herein further comprises at least one pump.

This embodiment has the advantage that the modification unit can be assembled without having to take gravity into consideration. In other words, without a pump the modification unit has to be designed such that gravity causes the product stream to flow through the various devices. Via employing a pump especially the use of a CFI as plug flow inverter is facilitated.

Moreover, it is preferred that the modification unit comprises several pumps. This would also allow an automatic control of the at least two valves and hence an automated process.

In a further embodiment the modification unit described herein comprises at least one waste outlet. Usually, a starting phase will be required prior to the production phase in which the modification unit generates the desired guidance-molecule linker complexes. During this starting phase material may already be generated which can be guided to the waste outlet. Hence, the waste outlet has the effect, that the parameters e.g. quality attributes of the guidance-molecule linker complexes are within a narrow range.

Moreover, a waste outlet generally offers the possibility to discard portions of the production stream. This is e.g. desirable, if process parameters such as pump rates etc. are not within a given relevant range. In such a case the portion of the production stream affected by the parameter being out of range can be discarded.

In another aspect the invention relates to a modular system for the continuous, germ-reduced production and/or processing of a guidance molecule drug conjugate, comprising the following units:
- at least one modification unit as described above,
- at least one unit for conjugation, comprising at least one mixing device, at least one plug flow reactor and at least one reservoir containing the biologically active substance
- at least one unit for concentration and re-buffering, comprising at least one buffer reservoir and at least one ultrafiltration device.
- least one unit for concentration and re-buffering, comprising at least one buffer reservoir and at least one diafiltration device
- at least one filter.

Preferably, said modular system for the continuous, germ-reduced production and/or processing of a guidance molecule drug conjugate comprises more than one filter.

Said filter preferably has a pore size of 0,1 µm - 4 µm, more preferably of 0,5 µm - 2,5 µm and most preferably of 0,5 µm - 2 µm.

Moreover, the modular system for the continuous, germ-reduced production and/or processing of a guidance molecule drug conjugate can also comprise the following units:
- at least unit for simultaneous modification and conjugation comprising at least one reservoir containing the guidance molecule in buffer solution and/or at least one inlet for a product stream containing the guidance molecule in buffer solution, at least one reservoir containing the linker solution and at least one reservoir containing the biologically active substance, at least one mixing device and at least one plug flow reactor
- at least one unit for concentration and re-buffering, comprising at least one buffer reservoir and at least one ultrafiltration device.
- at least one unit for concentration and re-buffering, comprising at least one buffer reservoir and at least one diafiltration device
- at least one filter.

It should be noted that instead of at least one reservoir containing the linker solution and at least one reservoir containing the biologically active substance, also at least one reservoir containing both the biologically active substance and the linker can be provided. In a preferred example of this embodiment, the biologically active substance and the linker are coupled before they are provided in said at least one reservoir containing both the biologically active substance and the linker.

Preferably, said modular system for the continuous, germ-reduced production and/or processing of a guidance molecule drug conjugate comprises more than one filter.

Said filter preferably has a pore size of 0,1 µm - 4 µm, more preferably of 0,5 µm - 2,5 µm and most preferably of 0,5 µm - 2 µm.

As used herein the term "modular system" is used interchangeably with the term "production plant" and refers to two or more units, which are connected to manufacture an ADC.

In a preferred embodiment the modular system described herein is a closed modular system.

Such a modular system which allows a continuous production of ADCs has the great advantage that it represents a closed system. Thus, the very hazardous active biological substance only has to be provided but thereafter is not handled anymore. Hence, *inter alia,* occupational safety is substantially improved.

As used herein the term "closed" means that the described modular system is operated in such a way that the fluid stream is not exposed to the room environment in order to minimize contamination of the product stream as well as exposure to the potentially hazardous active biological substance. Materials, objects, buffers, and the like can be added from outside, wherein, however, this addition takes place in such a way that exposure of the fluid stream to the room environment is avoided. Thus, sterile filters may be used to provide effective barriers from contaminants in the environment. Examples of closed systems include sterile single use bags supplied with integrated aseptic connection devices. Moreover, process systems may be opened but are "rendered closed" by a cleaning, sanitization and/or sterilization process that is appropriate or consistent with the process requirements, whether sterile, aseptic or low bioburden. Examples include process vessels that may be CIP'd and SIP'd between uses. Non-sterile systems such as chromatography or some filtration systems may also be rendered closed in low bioburden operations if appropriate measures are taken during the particular system setup. As used herein the term "closed" refers to both "functionally closed" as well as "closed" systems and as stated above generally refers to the fact that a described modular system is operated in such a way that the fluid stream is not exposed to the room environment

In one embodiment the modular system described herein is operated in a sterile environment. In this embodiment the modular system does not have to be closed in order to minimize contamination of the product stream.

Moreover, if suitable operation of the modular system described herein in a sterile environment can be combined with a closed operation of the modular system.

In a preferred embodiment the modular system is disposable and/or for single use.

Preferably the filter retains precipitations.

In one embodiment the closed modular system described herein comprises at least four 0,1µm filtration units and at least two 0,2 µm filtration units thereby allowing a parallel operation of the filtration units at the respective points of the closed modular system. Such a parallel operation is advantageous, since it allows the exchange of a blocked or damaged filtration unit while the product stream can pass through the other filtration unit. In other words, this embodiment facilitates a truly continuous process.

Maintenance of sterility is a challenge for a (semi)continuous production of antibodies and ADCs. Thus, preferably in the modification unit as well as in the modular system all components are connected together by tubes, in particular disposable tubes. The other components of the plant are also preferably disposable components; in particular, components selected from the group of disposable filtration elements, disposable valve tubing, disposable sensors (flow, pH, conductivity, UV, pressure), disposable tubes, disposable membranes, disposable connectors, disposable containers, disposable mixers and disposable sampling systems are used. Means for conveying liquid, in particular pumps, are also preferably disposable pumps.

In another aspect the invention relates to a method for the continuous, germ-reduced production and/or processing of a guidance molecule drug-conjugate, comprising the steps:
- providing an guidance molecule
- providing a linker,
- attachment of linker and guidance molecule,
- conjugation of the guidance molecule -linker complex to a biologically active substance,
- at least one continuous ultrafiltration of the product stream,
- at least one continuous diafiltration of the product stream,
- at least one filtration of the product stream .

Preferably the process is carried out in a closed manner.

The at least one filtration of the product stream is usually performed following the continuous diafiltration of the product stream. If it is beneficial to a given process other filtrations of the product stream can be carried out in addition or as alternative to said following the continuous diafiltration of the product stream.

Alternatively, the method for the continuous, germ-reduced production and/or processing of a guidance molecule drug-conjugate, can comprise the following steps:
- providing a guidance molecule
- providing a linker- biologically active substance substrate,
- conjugation of the guidance molecule to the linker- biologically active substance substrate,
- at least one continuous ultrafiltration of the product stream,
- at least one continuous diafiltration of the product stream,
- at least one filtration of the product stream,
wherein the process is carried out in a modular manner.

The at least one filtration of the product stream is usually performed following the continuous diafiltration of the product stream. If it is beneficial to a given process other filtrations of the product stream can be carried out in addition or as alternative to said following the continuous diafiltration of the product stream.

Alternatively, the method for the continuous, germ-reduced production and/or processing of a guidance molecule drug-conjugate, can comprise the following steps:
- providing a guidance molecule,
- providing a linker,
- providing a biologically active substance,
- joining of said guidance molecule, said linker and said biologically active substance to form a guidance molecule-linker-active substance conjugate,
- at least one continuous ultrafiltration of the product stream,
- at least one continuous diafiltration of the product stream,
- at least one filtration of the product stream,
wherein the process is carried out in a modular manner.

In other words, it is possible to first attach the linker to the guidance molecule or to first attach the linker to the biologically active substance or to simultaneously join all three components together.

The at least one filtration of the product stream is usually performed following the continuous diafiltration of the product stream. If it is beneficial to a given process other filtrations of the product stream can be carried out in addition or as alternative to said following the continuous diafiltration of the product stream.

In a further aspect the method for the continuous, germ-reduced production and/or processing of a guidance molecule-drug-conjugate described herein is used in a process for the continuous, germ-reduced production and/or processing of an antibody-drug-conjugate from a heterogeneous cell culture fluid mixture, comprising the steps:
- preparation of a particle-free fluid from a heterogeneous cell culture fluid mixture containing the antibody in the form of a product stream,
- at least one filtration,
- at least one chromatography step for cleaning the antibody comprising a cleaning via at least two chromatography columns and/or membrane adsorbers, respectively
- at least one viral clearance,
- at least one continuous ultrafiltration
- at least at least one continuous diafiltration of the product stream,
- processing the antibody derived after said continuous diafiltration using the methods described above to derive an antibody-drug-conjugate,
wherein the process is carried out in a continuous and modular manner

Thus a first step of said process for the continuous, germ-reduced production and/or processing of an antibody-drug-conjugate from a heterogeneous cell culture fluid mixture described above can be a continuous fermentation comprising a continuous cell retention to provide the heterogeneous cell culture fluid mixture containing the antibody and/or the provision of the heterogeneous cell culture fluid mixture containing the antibody, which was e.g. prepared using a batch or fed-batch approach.

Furthermore, a unit operation for formulation, e.g. adding of stabilizers and adjusting the pH of the final medicament, can be added downstream of the reservoir for storing the final product.

### Examples

### General considerations:

In order to provide an antibody-drug-conjugate under continuous, germ-reduced conditions in a modular process and preferably closed a production plant with the following units and accompanying process steps is constructed:
Unless otherwise specified, MasterFlex peristaltic pumps with an EasyLoad II pump head are used in the process. Masterflex or Cflex or Sanipure are used as tubes. All of the elements coming into contact with the product are gamma-irradiated with 25 kGy. In exceptional cases, if gamma irradiation is not allowable for reasons connected with materials technology, components are autoclaved at 121°C for at least 20 min. When possible, ready-to-use disposable articles ("disposables," "ready-to-use") are used as gamma-irradiated units. As a rule, all bags are connected to the units. Between each unit, a single-use gamma-irradiated bag is placed between the outlet flow of the unit n-1 and the inlet flow of the unit n as an equalizing tank. As a rule, there is an inlet and outlet flow at that time in each unit. In cases where venting of the product liquid is advantageous, the vessels are sealed off from the external environment via a 0.2 µm hydrophobic filter. Moreover, the process is controlled by a PCS7 process system.

A storage container comprising an antibody solution, e.g. an IgG is provided. Moreover, a storage bag comprising a linker solution is provided.

The antibody solution as well as the linker solution are pumped from their reservoirs, also termed storage bags, and allowed to mix and react, thereby modifying both components and generating an antibody-linker substrate. The conjugation is carried out in the next step, where, a solution comprising the biologically active substance, e.g. a solution comprising toxophores, is added to the fluid stream comprising the antibody-linker complex and all components are mixed, e.g. using a static mixer to generate the antibody-linker-toxophore conjugate. The toxophore load is measured via a UV-absorption at two specific wavelengths. The resulting product stream comprising antibody drug conjugates as well as unbound toxophores is optionally passed through a depth filter and then subjected to an ultrafiltration in order to concentrate the antibody. Then the product stream is subjected to a diafiltration e.g. the product stream is washed with a washing fluid via at least one capillary ultrafiltration membrane of a capillary ultrafiltration unit, wherein the product stream is conveyed into the capillary and the washing fluid is conveyed over the outside of the capillary and the product stream and the washing fluid are continuously fed into the capillary ultrafiltration unit, and are continuously removed from the capillary ultrafiltration unit and the product stream and the washing fluid are not circulated into the capillary ultrafiltration unit and the removal of the product stream is regulated such that no undesired net flows can pass from the capillary interior to the capillary exterior or vice versa and all fluid leaving the at least one capillary ultrafiltration unit in the direction of the product stream is passed through at least one guard purifier. For conveying the product stream (= feed stream) into the capillaries and the washing fluid (= permeate) on the outsides of the capillaries of the ultrafiltration unit in each case one pump is used. For the controlled removal of the product stream from the capillaries (= retentates), one further pump is used. Through the use of this pump (= retentate pump), control of the removal of the retentate is simplified. This is advantageous as there are no adequate flow sensors which reliably measure the low flow rates (≤ 100 ml/min) usually employed in this continuous process. Particularly preferably, peristaltic pumps can be used here with the advantage that peristaltic disposable pumps are commercially available, so that sterility and also disposable technology are available. Moreover, a pump is used for the controlled removal of the wash fluid (permeate).

Alternatively, instead of the continuous diafiltration described above a system comprising diafiltration slices in an ultrafiltration/diafiltration system can be used.

Finally the product stream is filtered using two 0.2 µm filters operated in parallel. Both the filter and the tube assembly are gamma-irradiated. The inlet and outlet lines are connected to gamma-irradiated bags) by means of sterile connectors, serving as an equalizing volume for fluctuating flow rates. For venting, the filters are coupled to hydrophobic 0.2 µm air filters, thus closing the unit within the meaning of the invention. The air filter is either an Emflon II from Pall Corp. or a Midisart 2000 from Sartorius Stedim. The venting valves are modified so that they are permeable even when closed, but still reliably sealed with respect to the environment.

### Specific example

The antibody solution, which had an initial concentration of 15,3 mg/mL was diluted with 100 mM potassiumphosphat (KPi)-Buffer (pH 7,5) to a final concentration of 10,72 g/L. The Linker-solution, here a SPDB Linker (N-succinimidyl-4-(2-pyridyldithio)butanoate) in DMA, was prepared with a concentration of 1,56 g/L.

These solutions were filled in the reservoirs and pumped with specific flow rates of 284,3 mL/h for AB-Solution and 20 mL/h for Linker-solution until the system reached a steady state. This period of time required by the system to reach a steady state, e.g. essentially constant pump rates, was termed start-up phase. In this example the start-up phase was defined as the first five residence times, where one residence interval equals the amount of time that a given portion of the product stream requires to flow through the modification unit. During start-up all produced material was directed to the waste outlet.

After the start-up phase the system was switched from start-up to production mode. Thus, the AB-Solution and the Linker-solution were pumped with specific flow rates of 284,3 mL/h for AB-Solution and 20 mL/h for Linker-solution into the mixing device, here a static Cascade- Micromixer from Ehrfeld was used which had the following setting [asymmetric Cascade mixer with max. inner diameter 150 µm, 11 mixing stages]. After passing the mixing device the product stream passed the plug flow reactor, here a Masterflex Tyron Tube was used with an inner-diameter of 3,1 mm having a total volume of 6,3 mL. Thereafter the product stream was collected in a reservoir, here a Schott-Flask with Bola-Connectors.

For collection of sample material the product stream was also directed to sample reservoirs. During the experiment samples of 20 mL were taken.

For process shut down, the flow was directed again to the waste outlet, the pumps were stopped and the all interconnected parts were flushed with solvent and water, which were filled into the reservoirs that previously contained the antibody and linkers solutions, respectively, for cleaning.

To prove that the modification reaction was successful under continuous process conditions it had to be demonstrated that the generated antibody-linker complexes had the same characteristics as antibody-linker complexes generated in a batch process. In order to do so the generated antibody-linker complexes were conjugated with a toxophore under standard conditions and their monomer content as well as their dimer content and the achieved Drug-to-Antibody ratio were determined.

For said analysis, 2,25 mL aliquot of the sample was diluted with 2 mL phosphate buffer and 0,209 mL of dimethylacetamide (DMA). Afterwards 40,9 µL of a 20 mM toxophore solution in DMA was added. After a reaction time of 18h the reaction was stopped by a buffer exchange to His/Gly (pH 5,5) by a PD10 column. Size exclusion chromatography was carried out and showed a monomer content of 96,4% and a dimer content of 2,8%. An aliquot of this sample was diluted and analyzed by UV-Measurement showing a Drug-to-Antibody-Ratio (DAR) of 3,4. The ULAR (Unconjugated Linker to Antibody Ratio) was determined to be below 0,03 using a specifc HPLC analysis, in which the sample is treated with a disulphide cleaving agent, the protein is removed and the HPLC detects the released mercaptopyridine.

In other words, it was shown that the continuous process conditions did not affect the stability of the final conjugate as otherwise the monomer content would be lower. Moreover, the antibody-linker complex generated under continuous conditions requires the same amount of toxophore per antibody-linker complex as in the batch process, since otherwise the DAR and ULAR values would be higher or lower.

Thus, overall these results demonstrated that the antibody-linker complexes generated in a continuous process using the modification unit as described herein are comparable to those generated in a batch process.

### Figures

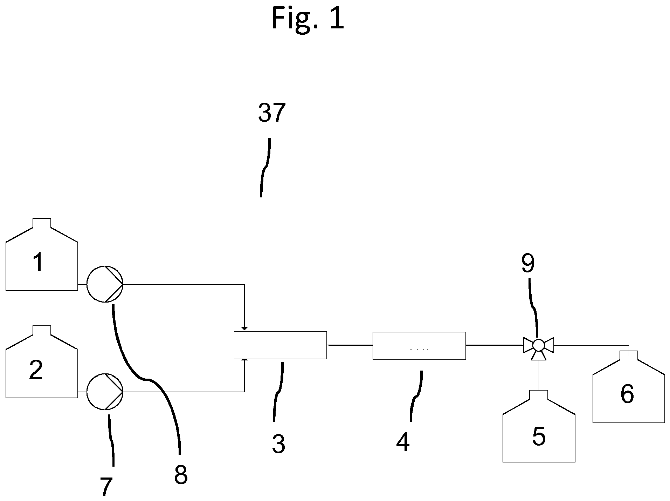

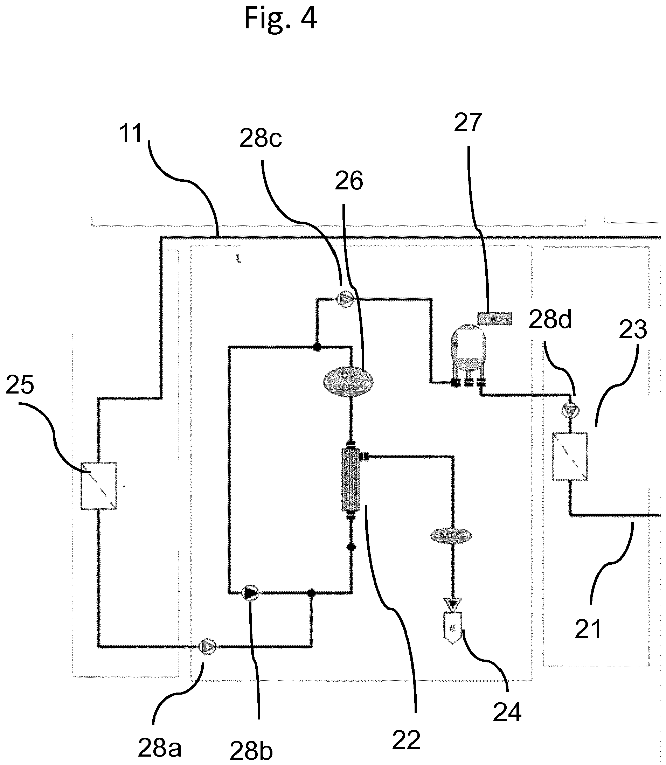

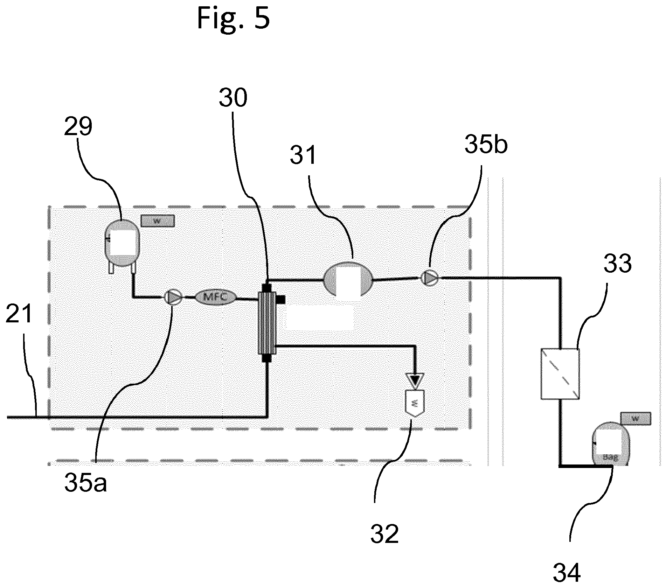

FIG 1 Overview of an example of a modification unit described herein for the continuous, germ-reduced processing of a guidance molecule and a linker comprising the following components: a reservoir containing the guidance molecule (1) in buffer solution, a reservoir containing the linker in solution (2), a mixing device (3), a plug flow reactor (4), a valve (7) for dosage of the guidance molecule, a valve (8) for dosage of the linker molecule, a reservoir for taking up the guidance molecule-linker complexes (5,a waste outlet (6) and a three way valve (9) for directing the product stream exciting the plug flow reactor (4) into the waste outlet (6) or the reservoir for taking up the guidance molecule-linker complexes (5). During operation, the guidance molecules in buffer solution and the linker solution are added in a controlled fashion via the two valves (7) and (8) to the mixing device (3).Following the mixing the product stream in this example flows through plug-flow reactor (4). Afterwards the product stream is collected in reservoir (5).

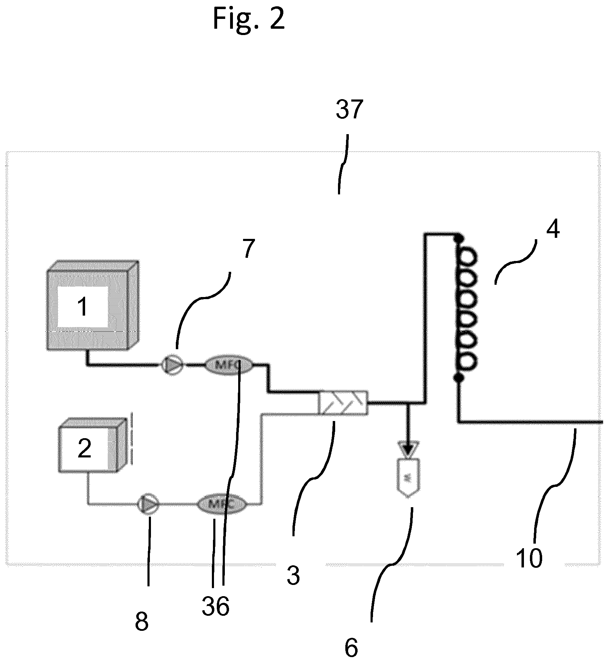

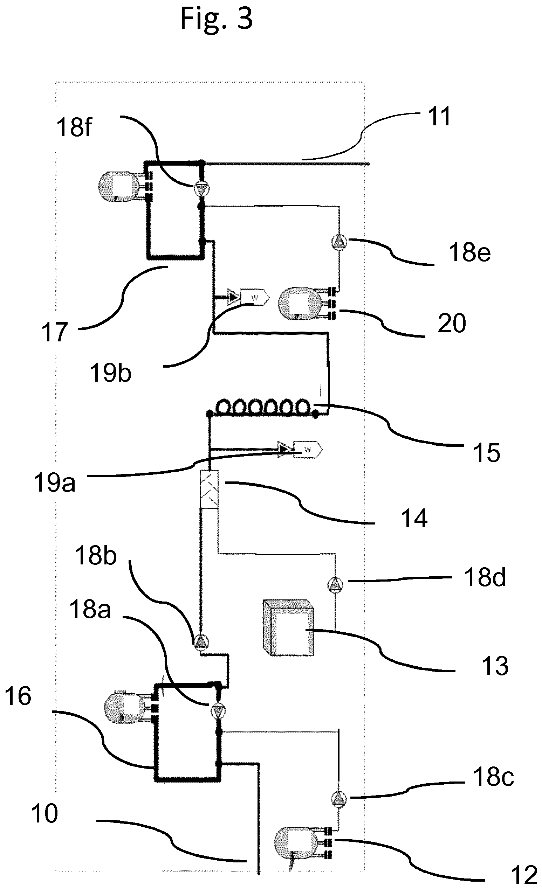

FIG 2 depicts a schematic illustration of an example of a modification unit described herein for the continuous, germ-reduced processing of a guidance molecule and a linker. In this example the modification unit is in fluid communication with a downstream unit. Hence, no reservoir for taking up the guidance molecule linker complexes is needed as the product stream continuously flows to the next unit operation. In this example the modification unit comprises the following components: a reservoir containing the guidance molecule (1) in buffer solution, a reservoir containing the linker in solution (2), a mixing device (3), a plug flow reactor (4), a valve (7) for dosage of the guidance molecule, a valve (8) for dosage of the linker molecule, a waste outlet (6) and a connection to the next unit operation (10). During operation, the guidance molecules in buffer solution and the linker solution are added in a controlled fashion via the two valves (7) and (8) to the mixing device (3).Following the mixing the product stream in this example flows through the plug-flow reactor (4). Afterwards the product stream continuously flows to the subsequent unit operation e.g. the one depicted in FIG. 3, via connection (10).
   It should be noted that intermediate reservoirs/storage containers (not depicted) may be included in the connection to the next unit operation (10), if this is beneficial.
   Moreover, it should be noted that as described above instead of providing an antibody in a reservoir (1) it could be provided from an upstream process continuously generating and purifying said antibody.
   Furthermore, in this example two optional mass flow controls (26) were employed.
FIG: 3 depicts a schematic overview of an exemplary unit operation carrying out the conjugation. In this example, said unit operation for conjugation comprises a connection to the previous unit operation (10) and a connection to the subsequent unit operation (11), a reservoir containing buffer solution (12), a reservoir containing a toxophore (13), a mixing device (14)- here a static mixer, a plug flow reactor (15), two homogenization loops (16) and (17) several valves (18a-f) for controlling the dosage of the different fluid streams, two waste outlets (19a and 19b) and a reservoir containing buffer solution (20) for the subsequent unit operation.
   During operation the product stream comprising the antibody-linker complexes generated in the previous unit operation, i.e. the unit for modification described herein, enters the unit operation for conjugation via connection (10) and buffer from reservoir (12) is added via the homogenization loop (16) before toxophore solution is dosed to the product stream containing the antibody-linker complexes from the reservoir containing the toxophore (13). Then the product stream comprising antibody-linker complexes and toxophore is mixed and following the mixing in device (14) the product stream in this example flows through the plug-flow reactor (15) and is optionally analyzed via UV-measurement. Afterwards buffer is added to the product stream from reservoir (20) via homogenization loop (17) in order to adjust the pH and the concentration and the antibody-linker-toxophore conjugates continuously flow to the subsequent unit operation e.g. the one depicted in FIG. 4.
FIG 4 depicts a schematic overview of an exemplary unit for continuous ultrafiltration comprising a connection to the previous unit operation (11) and a connection to the subsequent unit operation (21), an ultrafiltration device (22), a depth filter (23), a waste outlet (24), another filter (25), a detection device (26), a surge bag (27) and several valves (28a-d) for controlling the fluid flow.
   During operation the product stream comprising the antibody-linker-toxophore conjugates in this example enters this unit operation via connection (11), flows through filter (25), ultrafiltration device (22), detection device (26), surge bag (27) and depth filter (23) and then through connection (21) to the subsequent unit operation depicted in FIG. 5.
FIG 5: depicts a schematic overview of an exemplary unit for continuous dialysis comprising a connection from the previous unit operation (21), a reservoir containing buffer solution (29), a dialysis module (30), a detection device (31), a waste outlet (32), a filter (33), a reservoir for storing the final product (34), i.e the ultrafiltrated and dialysed antibody-linker-toxophore conjugates, as well as valves (35-b) for controlling the fluid stream.
   During operation the product stream comprising the antibody-linker-toxophore conjugates in this example enters this unit operation via connection (21) and flows through dialysis module (30), where buffer from reservoir (29) is exchanged for previous the buffer of the antibody-linker-toxophore conjugates. The previous buffer is discarded to waste outlet (32). The product stream comprising the antibody-linker-toxophore conjugates flows from the ultrafiltration module via the detection device (31) and the filter (33) to the reservoir for storing the final product (34).
   As described above unit operations for continuous cell culture generating the antibody, continuous cell separation as well as continuous purification of the antibody can be added upstream to enable a continuous production of an antibody linker conjugate starting with cell fermentation.

Furthermore, a unit operation for formulation, e.g. adding of stabilizers and adjusting the pH of the final medicament, can be added downstream of the reservoir for storing the final product.

### List of reference signs:

- 1.: reservoir containing the guidance molecule (1)
- 2.: reservoir containing the linker in solution (2),
- 3.: mixing device (3),
- 4.: plug flow reactor (4)
- 5.: reservoir (5)
- 6.: waste outlet (6)
- 7.: valve (7)
- 8.: valve (8)
- 9.: three way valve (9)
- 10.: connection between units (10).
- 11.: connection between units (11).
- 12.: reservoir containing buffer solution (12),
- 13.: reservoir containing a toxophore (13),
- 14.: mixing device (14)
- 15.: plug flow reactor (15),
- 16.: homogenization loop (16)
- 17.: homogenization loop (17)
- 18.: valves (18a-f)
- 19.: waste outlets (19a and 19b)
- 20.: reservoir containing buffer solution (20)
- 21.: connection between units (21)
- 22.: ultrafiltration device (22),
- 23.: depth filter (23),
- 24.: waste outlet (24),
- 25.: filter (25),
- 26.: detection device (26),
- 27.: surge bag (27)
- 28.: valves (28a-d)
- 29.: reservoir containing buffer solution (29),
- 30.: dialysis module (30),
- 31.: detection device (31),
- 32.: waste outlet (32),
- 33.: filter (33),
- 34.: reservoir for storing the final product (34)
- 35.: valves (35a and 35b)
- 36.: mass flow controls (36)
- 37.: modification unit (37)

## Claims

1. Modification unit (37) for the continuous, germ-reduced processing of a guidance molecule and a linker comprising the following components:
- at least one reservoir containing the guidance molecule in buffer solution (1) and/or at least one inlet for a product stream containing the guidance molecule in buffer solution,
- at least one reservoir containing the linker in solution (2),
- at least one mixing device (3),
- at least two valves (7,8), one for dosage of the guidance molecule and one for dosage of the linker molecule
- at least one outlet for the product stream comprising the guidance molecule-linker complexes and/or a reservoir for taking up the guidance molecule-linker complexes (5).

2. Modification unit according to claim 1, further comprising at least three valves.

3. Modification unit according to claims 1-2, further comprising at least one residence time device.

4. Modification unit according to anyone of the preceding claims, wherein the residence time device is a plug flow reactor (4).

5. Modification unit according to anyone of the preceding claims, wherein the guidance molecule is an antibody.

6. Modification unit according to anyone of the preceding claims, further comprising at least one pump.

7. Modification unit according to anyone of the preceding claims, further comprising at least one waste outlet (6).

8. Modular system for the continuous, germ-reduced production and/or processing of a guidance molecule drug conjugate, comprising the following units:
- at least one modification unit as claimed in claims 1-7,
- at least one unit for conjugation, comprising at least one a mixing device, at least one plug flow reactor and at least one reservoir containing the biologically active substance
- at least one unit for concentration and re-buffering, comprising at least one buffer reservoir and at least one ultrafiltration device.
- least one unit for concentration and re-buffering, comprising at least one buffer reservoir and at least one diafiltration device
- at least one filter.

9. Modular system according to claim 8, wherein the modular system is closed.

10. A method for the continuous, germ-reduced production and/or processing of a guidance molecule drug-conjugate, comprising the steps:
- providing an guidance molecule
- providing a linker,
- attachment of linker and guidance molecule,
- conjugation of the guidance molecule -linker complex to a biologically active substance,
- at least one continuous ultrafiltration,
- at least one continuous diafiltration of the product stream,
- at least one filtration of the product stream
wherein the process is carried out in a modular manner.

11. Method according to claim 10, wherein the method is carried out in a closed manner.

12. Use of the method according to claims 10-11 in a process for the continuous, germ-reduced production and/or processing of an antibody-drug-conjugate from a heterogeneous cell culture fluid mixture, comprising the steps:
- preparation of a particle-free fluid from a heterogeneous cell culture fluid mixture containing the antibody in the form of a product stream,
- at least one filtration,
- at least one chromatography step for cleaning the antibody comprising a cleaning via at least two chromatography columns and/or membrane absorbers, respectively
- at least one viral clearance,
- at least one continuous ultrafiltration
- at least at least one continuous diafiltration,
- processing the antibody derived in said continuous diafiltration using the method of claims 10-11 to derive an antibody-drug-conjugate wherein the process is carried out in a continuous, closed and modular manner.
